# EUROPEAN PATENT APPLICATION

(11) **EP 3 133 062 A1**
(43) Date of publication of application: **22.02.2017**
(21) Application number: 15460045.6
(22) Date of filing: 19.08.2015
(51) Int. Cl.: C07D 219/06, C07D 405/04, C07D 239/60, C07C 15/00, C07F 9/24

(54) **PROCESS FOR THE PREPARATION OF A PHARMACEUTICAL AGENT**

(71) Applicant: Zaklady Farmaceutyczne Polpharma SA, 83-200 Starogard Gdanski (PL)
(72) Inventor: KOSIK, Kamil, 11-400 Ketrzyn (PL); SAGOL, Karol, 83-110 Tczew (PL); GODZISZ, Mateusz, 59-900 Zgorzelec (PL)
(74) Representative: Obrycki, Pawel Andrzej

(57) **Abstract**

This invention provides a process for providing phosphoramidate derivatives, comprising performing a nucleophilic substitution reaction at phosphorous, where a phosphoryl chloride derivative is reacted with an oxime derivative to provide activated phosphoramidate intermediates, the intermediates find utility in a second nucleophilic substitution reaction where an activated phosphoramidate is reacted with a primary alcohol-containing sugar derivative, wherein the product of the reaction is sofosbuvir.

## Description

The present invention relates to a process for the preparation of a pharmaceutical agent, and particularly to a process for preparing sofosbuvir.

Hepatitis C virus (HCV) represents a major global health problem with approximately 200 million people infected worldwide. The virus can cause either an acute or chronic hepatitis infection, which can range in severity from a mild and short-lived illness to a serious lifelong condition. Acute HCV infection is often asymptomatic, and is only rarely associated with life-threatening disease. Approximately 15-45% of infected persons spontaneously clear the virus within six months and without treatment. Moreover, approximately 55-85% will progress to, and suffer from, a chronic disease state. Chronically infected individuals typically ultimately present with either liver cirrhosis or hepatocellular carcinoma.

HCV is a bloodborne virus and hence the route of transmission is effectively limited to parenteral modes. The World Health Organisation lists injecting drug use through the sharing of needles, inadequate sterilisation of medical equipment and transfusion of unscreened blood as the most common modes of transmission. The virus is also known to be transmitted sexually, and can be passed from mother to baby however these modes are less common.

The current standard of care for HCV infected patients is restricted to immunotherapy with pegylated interferons (IFNs) alone, or in combination with the nucleoside analog ribavirin. It is generally accepted that these approaches are of limited clinical benefit. Moreover, interferon immunotherapy provides a number of undesirable side effects, which prevent a significant number of HCV-infected individuals from receiving antiviral therapy. The elimination of IFNs from treatment regimens is therefore highly desirable.

Sofosbuvir is named (S)-isopropyl 2-((*S*)-(((2*R*,3*R*,4*R*,5*R*)-5-(2,4-dioxo-3,4-dihydropyrimidin-1(2H)-yl)-4-fluoro-3-hydroxy-4-methyltetra-hydrofuran-2-yl)methoxy)-(phenoxy)phosphorylamino)propanoate. The first (S) in formula denotes the stereochemistry of the chiral carbon atom contained within the alanine moiety and the second (S) at the chiral phosphorous atom. Sofosbuvir is represented structurally by the formulae below (Haworth projection and structural formula, respectively). 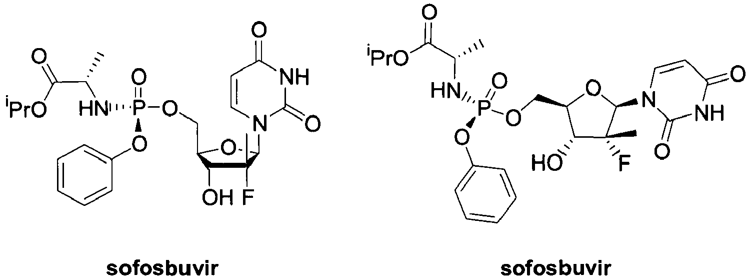 Sofosbuvir is indicated for the treatment of patients presenting with chronic hepatitis C (of genotypes 1 to 6). The drug is used in combination with pegylated interferon and ribavirin, or ribavirin alone. It has also been dosed in combination with ledipasvir in an interferon-free combination for the treatment of genotype 1 chronic hepatitis C. Sofosbuvir is a prodrug and is metabolised *in vivo* to the active antiviral 2'-deoxy-2'-α-fluoro-β-*C*-methyluridine-5'-triphosphate. The triphosphate is a substrate for non-structural protein 5B (NS5B) a HCV viral RNA polymerase. Therefore the triphosphate directly inhibits NS5B, and consequently, the replication and synthesis of viral RNA.

Sofosbuvir is commercially marketed by Gilead Sciences Ltd under the trade name Sovaldi and is formulated as an oral film-coated tablet in an amount of 400 mg. The drug is also marketed by Gilead Sciences Ltd in combination with ledipasvir under the trade name Harvoni®, wherein the formulation is provided as an oral film-coated tablet containing 400 mg of sofosbuvir and 90 mg of ledipasvir. In both instances sofosbuvir is marketed as the (S)-diastereoisomer (with respect to the stereochemistry of the phosphorous atom).

EP 2 203 462 details a synthesis of a methyl ester analogue of sofosbuvir:

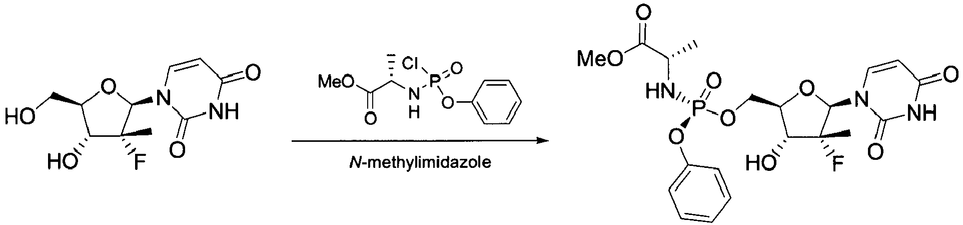

Sofosbuvir is an asymmetrically substituted phosphoramidate which contains five chiral centres (four of which are contiguous) in addition to a chiral phosphorous atom. A complicating factor in the synthesis of sofosbuvir and more broadly with compounds of a complex stereochemical nature, is the maintenance of the integrity of each defined stereocentre. Any erosion or complete loss of stereochemical information can normally be counteracted by repeated purification (i.e. to recover and afford enantiomerically or diastereomerically enriched compositions) but the additional purification steps increase overall production costs and are generally unsatisfactory. Furthermore, it is also desirable to provide stereoselective chemical transformations. That is, where a stereocentre can be generated with bias, at a location wherein prior to a reaction no stereocentre was defined, or existed - for example, at a prochiral phosphorous atom.

Therefore there remains a need in the art for improving both the overall synthesis of such entities and the individual constituent transformations (step-to-step efficacy) within the syntheses.

Accordingly the present invention provides a process for preparing a compound of formula I 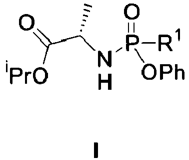 comprising reacting a compound of formula II 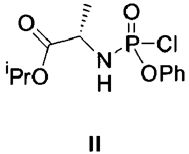 with a compound of formula IIIa, IIIb or IIIc 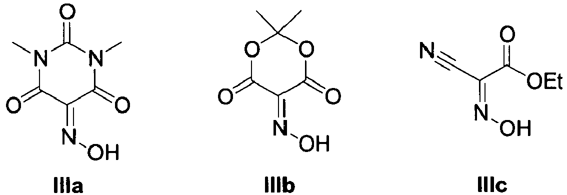 wherein R¹ represents:

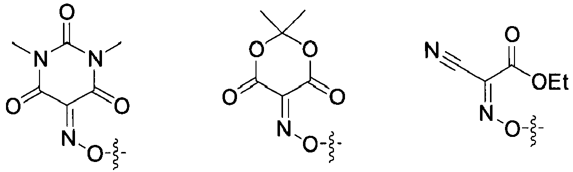

There is herein provided a process for the synthesis of activated phosphoramidate derivatives and processes for their use in the (stereoselective) synthesis of sofosbuvir.

In accordance with the present invention, there is provided a process for the preparation of a compound of formula I. The process comprises a single step, wherein a compound of formula II is reacted with a compound of formula IIIa, IIIb or IIIc. The step comprises a nucleophilic substitution reaction at phosphorous, wherein the chlorine atom present in the compound of formula II is displaced by an oxime-containing compound selected from compounds of formula IIIa, IIIb, or IIIc.

It is preferred wherein a compound of formula IIIa, IIIb or IIIc is reacted with a compound of formula II.

It is most preferred where a compound of formula IIIa is reacted with a compound of formula II.

It is also preferred wherein a compound of formula IIIa, IIIb or IIIc is reacted with a compound of formula II in the presence of a base, for example a trialkylamine base. It is more preferred where a compound of formula IIIa is reacted with a compound of formula II in the presence of a base, for example a trialkylamine base. It is most preferred where a compound of formula IIIa is reacted with a compound of formula II in the presence of a base, for example a trialkylamine base, wherein the trialkylamine base is triethylamine.

In accordance the present invention, the process for preparing a compound of formula I is conducted in a solvent (i.e. an organic solvent) or a mixture of solvents. It is known in the art which particular solvent is most appropriate and most effective for each category of chemical transformation. For example, water- or air-sensitive transformations require the use of non-aqueous, non-protic solvents such as acetonitrile, tetrahydrofuran, dioxane, dimethylsulfoxide dichloromethane, chloroform or N-methylpyrrolidine. Furthermore, water- or air-sensitive reactions may also require an atmosphere of inert gas (to prevent atmospheric water from compromising a transformation) typical inert gases include nitrogen and argon. Alternatively, reactions requiring the use of an aqueous reagent (e.g. aqueous hydrochloric acid or aqueous sodium hydroxide) may require the use of one or more organic co-solvents. In this context, an organic co-solvent is added to aid the dissolution, or partial dissolution of a less polar organic reagent. Typical organic co-solvents for use with such aqueous reagents include, toluene, alcohols (methanol, ethanol, isopropanol), tetrahydrofuran and dimethylsulfoxide. The only limitation in regard to reaction efficacy when using organic co-solvents with aqueous solvents is the requirement for some degree of miscibility of the organic co-solvent with water. To aid problems with miscibility and or reaction efficacy a phase transfer catalyst is employed (e.g. tetrabutylammonium bromide).

Preferred solvents for use in preparing a compound of formula I are aprotic organic solvents. The term for use in preparing should be understood as the solvent in which the reactive transformation occurs. The solvent could therefore equally be termed the reaction solvent. Typical examples include dichloromethane, chloroform, tetrahydrofuran, 2-methyltetrahydrofuran, toluene, hexanes and ethyl acetate. It is particularly preferred where the solvent is dichloromethane or tetrahydrofuran.

It should also be noted, that where practically possible and in accordance with the present invention, processes are ideally conducted at room temperature. Any heating (above room temperature (25°C)) or cooling (below room temperature (25°C)) leads to a substantial increase in operating costs (especially when processes are performed on large or industrial scale - multi-kilogram or multi-tonne). It is apparent that the greater the deviation away from room temperature (above or below) the greater the operating costs. Therefore the achievement of any reduction of a traditionally high-operating temperature, or increase in a traditionally low-operating temperature reaction is particularly preferred.

The process for preparing a compound of formula I is typically performed at a temperature of from -80°C to 15°C and preferably at a temperature of from -70°C to -15°C. It is most preferred wherein the process is performed at a temperature of from -10°C to 15°C.

It is therefore preferred wherein a compound of formula IIIa, IIIb or IIIc is reacted with a compound of formula II in the presence of a base, for example a trialkylamine base. It is more preferred where a compound of formula IIIa is reacted with a compound of formula II in the presence of a base, for example a trialkylamine base. It is most preferred where a compound of formula IIIa is reacted with a compound of formula II in the presence of a base, for example a trialkylamine base, wherein the trialkylamine base is triethylamine and wherein the process is performed at a temperature of from -80°C to 15°C and preferably at a temperature of from -70°C to -15°C. It is most preferred wherein the process is performed at a temperature of from -10°C to 15°C.

Purification of a compound of formula I is preferably simplified following the removal of the reaction solvent by, for example, reduced pressure evaporation or distillation. Upon removal of the reaction solvent a further solvent is added to the reaction vessel or residue. The further solvent preferably comprises a solvent in which hydrochloride salts of organic bases, for example organic amine bases, preferably organic alkylamine bases, for example trialkylamines (trimethylamine, triethylamine, diisopropylethylamine) hydrochloride, *N*-methylmorpholine hydrochloride and aryl amine bases, for example pyridine hydrochloride are insoluble. Precipitation of trialkylamine hydrochloride salts using an appropriate organic solvent allows for the avoidance of time consuming and potentially product-losing aqueous washing steps. Preferred solvents used for the precipitation of trialkylamine hydrochloride salts include tetrahydrofuran, ethyl acetate, diethyl ether and methyl *tert*-butyl ether. A particularly preferred solvent is tetrahydrofuran.

Purification of a compound of formula I is further preferably simplified following the removal of trialkylamine hydrochloride salts, wherein the filtrate solvent is typically removed by, for example, reduced pressure evaporation or distillation. Upon removal of the filtrate solvent a further solvent is added to the reaction vessel or residue, wherein addition of the solvent allows precipitation of a compound of formula I. Preferably a non-polar aprotic solvent is added to the reaction vessel or residue, for example n-heptane, hexane, cyclohexane, toluene, methyl *tert*-butyl ether or pentane, most preferably n-heptane or methyl *tert*-butyl ether is added.

In accordance with the present invention there is also provided a compound of formula Ia, Ib or Ic,

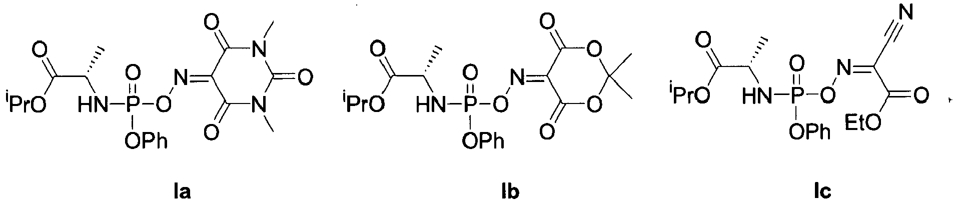

The compounds of formula Ia, Ib or Ic are provided following the reaction of a compound of formula II with a compound of formula IIIa, IIIb or IIIc respectively. Compounds Ia, Ib and Ic represent activated phosphoramidate derivatives, and find utility in the synthesis of sofosbuvir and in the stereoselective synthesis of sofosbuvir.

It is preferred wherein a compound of formula IIIa, IIIb or IIIc is reacted with a compound of formula II. It is most preferred where a compound of formula IIIa is reacted with a compound of formula II. It is even more preferred wherein a compound of formula la is provided following the reaction of a compound of formula IIIa with a compound of formula II.

In an embodiment of the present invention, the reaction proceeds with stereoselectivity in favour of the desired configuration at the phosphorous atom. Thus, compounds of formula Ia, Ib or Ic can be provided as a mixture of a diastereoisomers. The diastereoisomers are compounds of formula (S)-Ia and (*R*)-Ia, or (*S*)-Ib and (*R*)-Ib, or (*S*)-Ic and (*R*)-Ic.

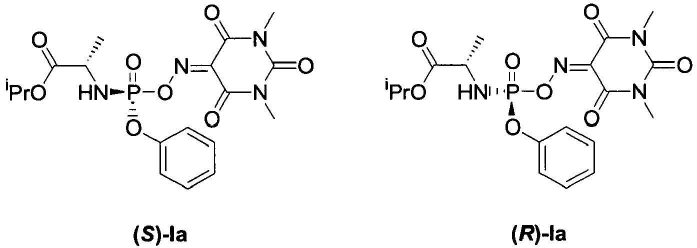

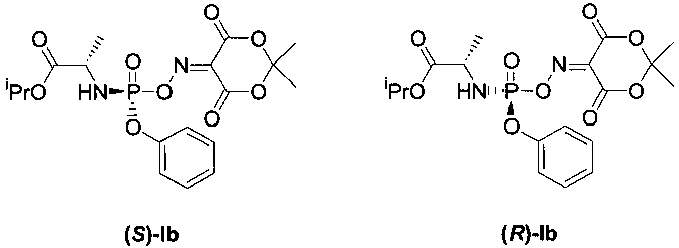

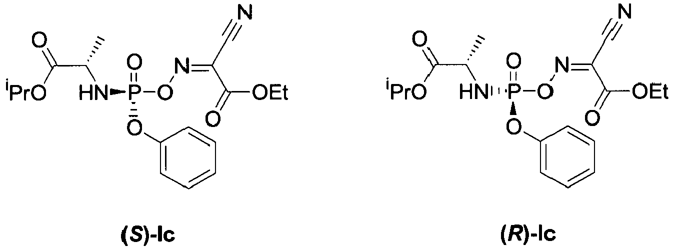

The diastereoisomers as referred to hereinabove are denoted by (S) or (R) nomenclature, wherein the (S) or (R) notation refers to the stereochemistry at the phosphorous centre.

The compounds of formula la, Ib or Ic display excellent solubility in a range of organic solvents, for example tetrahydrofuran and dichloromethane, which are advantageous to their use in synthesis.

The present invention also provides a process for preparing sofosbuvir, 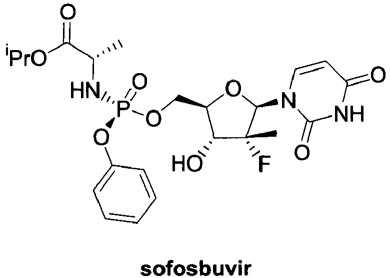 comprising, reacting a compound of formula I and a compound of formula IV, 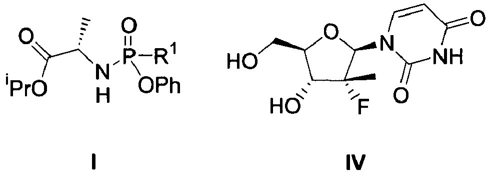 wherein R¹ is as defined hereinabove:
In accordance with the present invention, there is provided a process for the preparation of sofosbuvir. The process comprises a single step, wherein a compound of formula I is reacted with a compound of formula IV. The step comprises a nucleophilic substitution reaction at phosphorous, wherein an oxime-containing moiety represented by R¹, is displaced by a compound of formula IV, to provide sofosbuvir.

Sofosbuvir is provided in a stereoselective manner. It is provided as a mixture of diastereoisomers, wherein the diastereomeric ratio (with respect to the stereochemistry at phosphorous) is 10:1 or greater, in favour of sofosbuvir (the (S)-diastereoisomer), more preferably the diastereomeric ratio is 15:1 or greater in favour of sofosbuvir (the (S)-diastereoisomer). The S:R diastereoisomeric products are exemplified by compounds sofosbuvir and (R)-diastereoisomer respectively.

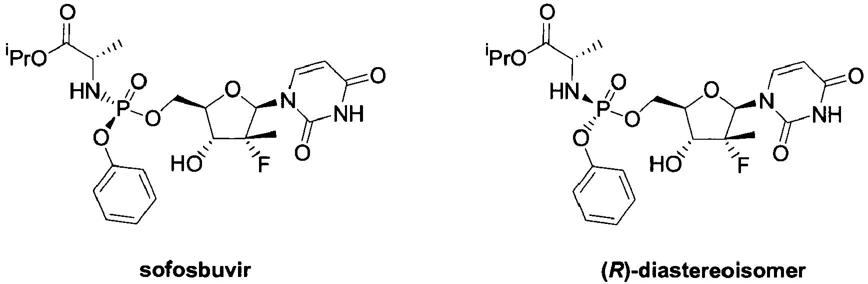

Diastereomeric ratios can be determined by methods known in the art. In accordance with the present invention, the most preferred method for determining the diastereomeric ratio is HPLC (high-performance liquid chromatography). Preferred solvents by HPLC are H₂O/MeCN (water/acetonitrile). Typically, HPLC analysis can be performed using a 150 x 4.6 mm Waters XBridge Shield RP-18 column, where detection is set at 200-400nm, and wherein elution is conducted using a H₂O/MeCN gradient (90/10 to 10/90) over a period of 20 minutes. With reference to purification of the examples, Sp notation denotes sofosbuvir and Rp notation denotes the (R)-diastereoisomer.

Furthermore, the diastereomeric ratio (i.e. the ratio of sofosbuvir to (R)-diastereoisomer) may be improved by a subsequent purification step. Steps to improve diastereomeric ratio are known in the art, and typically include selective crystallisation and recrystallisation processes.

In accordance with the present invention there is also provided a process for preparing sofosbuvir comprising preparing a compound of formula I and reacting a compound of formula I and a compound of formula IV. The process comprises the two independent nucleophilic substitution reactions at the phosphorous centre, first at the phosphorous centre of a compound of formula II and second at the phosphorous centre of a compound of formula I. The reactions are preferably conducted independent of one another (i.e. the reactions are performed as two individual steps, and not in "one-pot" or via a single chemical transformation).

It is also provided that following the process for preparing a compound of formula I and the process for preparing sofosbuvir, sofosbuvir is crystallised, for example using the conditions described in WO2010135569.

In accordance with the present invention, the process for preparing sofosbuvir is conducted in a solvent (typically an organic solvent) or a mixture of solvents. It is known in the art which particular solvent is most appropriate and most effective for each category of chemical transformation. For example, water- or air-sensitive transformations require the use of non-aqueous, non-protic solvents such as acetonitrile, tetrahydrofuran, dioxane, dimethylsulfoxide dichloromethane, chloroform or N-methylpyrrolidine. Furthermore, water- or air-sensitive reactions may also require an atmosphere of inert gas (to prevent atmospheric water from compromising a transformation) typical inert gases include nitrogen and argon. Alternatively, reactions requiring the use of an aqueous reagent (e.g. aqueous hydrochloric acid or aqueous sodium hydroxide) may require the use of one or more organic co-solvents. In this context, an organic co-solvent is added to aid the dissolution, or partial dissolution of a less polar organic reagent. Typical organic co-solvents for use with such aqueous reagents include, toluene, alcohols (methanol, ethanol, isopropanol), tetrahydrofuran and dimethylsulfoxide. The only limitation in regard to reaction efficacy when using organic co-solvents with aqueous solvents is the requirement for some degree of miscibility of the organic co-solvent with water. To aid problems with miscibility and or reaction efficacy a phase transfer catalyst is employed (e.g. tetrabutylammonium bromide).

Preferred solvents for use in preparing sofosbuvir are aprotic organic solvents. The term for use in preparing should be understood as the solvent in which the reactive transformation occurs. The solvent could therefore equally be termed the reaction solvent. Particularly preferred solvents are tetrahydrofuran and 2-methyltetrahydrofuran.

The oxime-containing compounds of formula IIIa, IIIb and IIIc are known in the art. A compound of formula IIIa used in conjunction with the present invention is also known as Oxyma-B. The compound of formula IIIb used in conjunction with the present invention is also known as isonitroso Meldrum's acid or (HONM) and the compound of formula IIIc used in conjunction with the present invention is also known as Oxyma.

The process for preparing sofosbuvir can be carried out at a temperature of from -80 to 65°C, preferably at a temperature of from -40°C to 50°C, more preferably at a temperature of from -10°C to 0°C.

The process for preparing sofosbuvir can additionally comprise a base. The base is an organic base, preferably an alkyl magnesium-containing base, for example *tert*-butylmagnesium chloride, isopropylmagnesium chloride, isopropylmagnesium chloride/lithium chloride complex, methylmagnesium chloride, butylmagnesium chloride and isobutylmagnesium chloride. It is preferred wherein the base is *tert*-butylmagnesium chloride.

It is particularly preferred wherein the base is turbo Grignard (which is also known as isopropylmagnesium chloride/lithium chloride complex). Lithium chloride complexes of alkyl magnesium-containing bases often increase the reactivity of the bases per se. The lithium chloride disrupts the polymeric aggregation of alkyl magnesium chlorides and also increases the polarisation of the alkyl-magnesium bond, which enhances the reactivity of the magnesium centre and hence the reactivity of the base.

In accordance with the present invention it is therefore preferred wherein, the process for preparing sofosbuvir can be carried out at a temperature of from -80 to 65°C, preferably at a temperature of from -40°C to 50°C, more preferably at a temperature of from -10°C to 0°C and preferably in the presence of a base, wherein the base is an organic base, preferably an alkyl magnesium-containing base and most preferably turbo Grignard (which is also known as isopropylmagnesium chloride/lithium chloride complex) wherein the preferred embodiments provide a diastereomeric ratio is 15:1 or greater, in favour of sofosbuvir.

If necessary, further steps may be included to provide sofosbuvir in the form of a salt, ester, solvate or complex.

It can be seen that the present invention provides a process for providing phosphoramidate derivatives, comprising performing a nucleophilic substitution reaction at phosphorous, where a phosphoryl chloride derivative is reacted with an oxime derivative to provide activated phosphoramidate intermediates, the intermediates find utility in a second nucleophilic substitution reaction where an activated phosphoramidate is reacted with a primary alcohol-containing sugar derivative, wherein the product of the reaction is sofosbuvir.

The present invention will now be described with reference to the examples, which are not intended to be limiting.

### Examples

### Example 1

### Propan-2-yl-N-[{[(1,3-dimethyl-2,4,6-trioxotetrahydropyrimidin-5(2H)-ylidene)amino]oxy}(phenoxy) phosphoryl]-L-alaninate

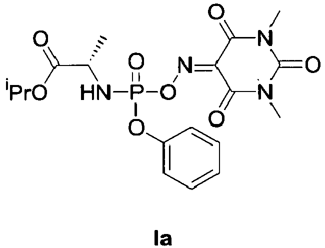

A stirred solution of 2 g (9.5 mmol) of phenyl dichlorophosphate in 30 mL of CH₂Cl₂ was cooled to 0°C. Next, 1.6 g (9.5 mmol) of L-alanine isopropyl ester hydrochloride in 20 mL of CH₂Cl₂ was added in one portion. The reaction mixture was stirred for 10 minutes. 1.92 g (19 mmol) of triethylamine diluted by 5 mL of CH₂Cl₂ was added dropwise during 10 minutes. The obtained suspension was stirred for 1 h at 0°C.

Oxyma-B, 1.76 g (9.5 mmol), in 20 mL of CH₂Cl₂ was then added and reaction mixture was stirred for 5 minutes. 0.96 g (9.5 mmol) of triethylamine diluted by 5 mL of CH₂Cl₂ was added during 10 minutes and reaction mixture was stirred for 10 minutes at 0°C. The mixture was allowed to warm to 20-25°C and stirred for 1 h at 20-25°C. Solvents were distilled under reduced pressure and to the residue was added 30 mL of diethyl ether. Obtained suspension was stirred for 10 minutes and the triethylamine hydrochloride solid was filtered. Filtrate was concentrated under reduced pressure. To the obtained residue 20 mL of ethyl acetate:hexane (1:4 v/v) mixture was added and the reaction mixture was stirred for 1 h at 20-25°C. Obtained solid was filtered and washed with 5 mL of ethyl acetate:hexane (1:4 v/v) mixture. The product was dried under reduced pressure.

Obtained was 0.7 g (16.2%) of a pale-pink solid; Isomer B - ¹H NMR (d₆ DMSO) δ 7.40 (dd, J = 8.8, 7.4, 2H), 7.23 (d, J = 8.8, 2H), 7.22 (t, J = 7.4, 1H), 6.81 (dd, J = 14.8, 10.2, NH), 4.83 (heptet, J = 6.2, 1H), 3.88 (m, 1H), 3.21 (s, 3H), 3.16 (s, 3H), 1.30 (d, J = 6.9, 3H), 1.13 (d, J = 6.3, 6H); Isomer C - ¹H NMR (d₆ DMSO) δ 7.40 (t, J = 8.1, 2H), 7.31 (d, J = 8.4, 2H), 7.23 (t, J = 7.4, 1H), 6.71 (dd, J = 12.9, 9.9, NH), 4.88 (heptet, J = 6.2, 1H), 3.91 (m, 1 H), 3.20 (s, 3H), 3.15 (s, 3H), 1.29 (d, J = 6.1, 3H), 1.18 (d, J = 6.3, 3H), 1.16 (d, J = 6.3, 3H); HRMS (isomers B and C) m/z [TOF MS] found 477.1144 [M + Na]⁺, C₁₈H₂₃N₄O₈PNa calcd 477.3606.

### Example 2

### Sofosbuvir

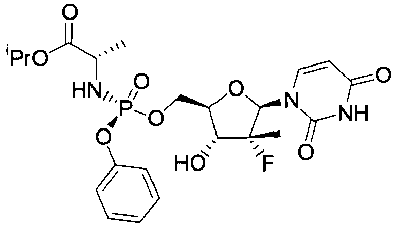

2'-Deoxy-2'-fluoro-2'-C-methyluridine, 0.14 g (0.55 mmol), was dissolved in 20 mL of anhydrous THF and cooled to 0°C. Added was 1.1 ml (1.1 mmol) of *^{t}*BuMgCl solution (1 M solution in THF) and the obtained suspension was stirred at 0°C for 10 minutes. Next, added was 0.5 g of Oxyma-B intermediate (compound of formula la) (1.1 mmol) and the reaction mixture was stirred for 1 h at 0°C. The mixture was then warmed to 20-25°C during 2 h and stirred for 15 h at 20-25°C. After reaction, 40 mL of ethyl acetate and 20 mL of saturated ammonium chloride solution was added. After stirring for 15 minutes the phases were separated. The organic phase was washed twice with 20 mL of 0.5 N HCl, twice with 20 mL of saturated NaHCO₃ solution and with 20 mL of brine solution. The organic phase was dried over anhydrous MgSO₄ and concentrated under reduced pressure. The product was obtained as a mixture of isomers (Sp:Rp isomers - 2.5:1). The isomeric ratio was determined by HPLC (150 x 4.6 mm Waters XBridge Shield RP-18 column, detection at 200-400nm, H₂O/MeCN gradient 90/10 to 10/90 at 20 minutes, Sp R_{T} = 10.3 min and Rp R_{T} = 10.1 min).

### Example 3

### Propan-2-yl-N-[{[(1,3-dimethyl-2,4,6-trioxotetrahydropyrimidin-5(2H)-ylidene)amino]oxy}(phenoxy) phosphoryl]-L-alaninate

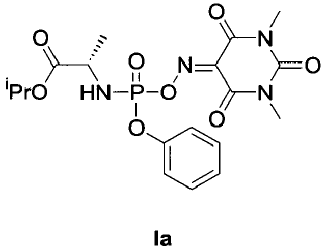

A stirred solution of 4 g of phenyl dichlorophosphate (19 mmol) in 40 mL of CH₂Cl₂ was cooled to 0°C. Next, 3.2 g (19 mmol) of L-alanine isopropyl ester hydrochloride in 30 mL of CH₂Cl₂ was added in one portion. Reaction mixture was stirred for 10 minutes. 3.84 g (38 mmol) of triethylamine diluted by 10 mL of CH₂Cl₂ was added dropwise during 10 minutes. The obtained suspension was stirred for 1 h at 0°C. Then, 3.52 g (19 mmol) of Oxyma-B in 30 mL of CH₂Cl₂ was added and reaction mixture was stirred for 5 minutes. 1.92 g (19 mmol) of triethylamine diluted by 10 mL of CH₂Cl₂ was added during 10 minutes and reaction mixture was stirred for 10 minutes at 0°C. The mixture was then heated slowly to 20-25°C and stirred for 1 h at this temperature. Solvents were distilled under reduced pressure and to the residue was added 60 mL of ethyl acetate. Obtained suspension was stirred for 10 minutes and the triethylamine hydrochloride solid was filtered. Filtrate was concentrated under reduced pressure. To the obtained residue 45 mL of ethyl acetate was added and suspension was heated to 40°C to dissolution. Obtained solution was cooled to 0°C, 35 mL of hexane was added during 20 minutes and suspension was stirred for 1 h at 0°C. Solid was filtered and washed twice with 15 mL of hexane. The product was dried under reduced pressure to give 3.3 g of desired product. Yield: 38.2%.

### Example 4

### Sofosbuvir

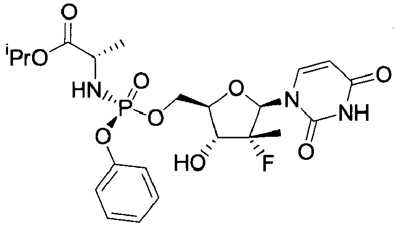

2'-Deoxy-2'-fluoro-2'-C-methyluridine, 0.23 g (0.88 mmol), was dissolved in 30 mL of anhydrous THF and cooled to 0°C. Added was 1.76 mL (1.76 mmol) of *^{t}*BuMgCl solution (1 M solution in THF) and the obtained suspension was stirred at 0°C for 10 minutes. Next, added was 0.8 g (1.76 mmol) of Oxyma-B intermediate (compound of formula la) and the reaction mixture was stirred for 1 h at 0°C. The mixture was then warmed to 20-25°C during 2 h and stirred for 15 h at 20-25°C. After reaction 60 mL of ethyl acetate and 30 mL of saturated ammonium chloride solution were added. After stirring for 15 minutes the phases were separated. The organic phase was washed twice with 30 mL of 0.5 N HCl, twice with 30 mL of saturated NaHCO₃ solution and with 30 mL of brine solution. The organic phase was dried over anhydrous MgSO₄ and concentrated under reduced pressure. The product was obtained as a mixture of isomers (Sp:Rp isomers - 9:1). The isomeric ratio was determined by HPLC (150 x 4.6 mm Waters XBridge Shield RP-18 column, detection at 200-400nm, H₂O/MeCN gradient 90/10 to 10/90 at 20 minutes, Sp R_{T} = 10.3 min and Rp R_{T} = 10.1 min).

### Example 5

### Propan-2-yl-N-[{[(1,3-dimethyl-2,4,6-trioxotetrahydropyrimidin-5(2H)-ylidene)amino]oxy}(phenoxy) phosphoryl]-L-alaninate

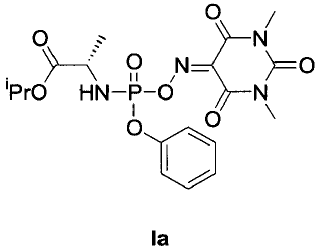

A stirred solution of 11.4 g of phenyl dichlorophosphate (54 mmol) in 80 mL of CH₂Cl₂ was cooled to 0°C. Next, 9.05 g (54 mmol) of L-alanine isopropyl ester hydrochloride in 50 mL of CH₂Cl₂ was added in one portion. Reaction mixture was stirred for 10 minutes. 10.94 g (108 mmol) of triethylamine diluted by 15 mL of CH₂Cl₂ was added dropwise during 10 minutes at 0°C. The obtained suspension was stirred for 1 h at 0°C. 15 g (54 mmol) of Oxyma-B in 40 mL of CH₂Cl₂ was added and reaction mixture was stirred for 5 minutes. 5.47 g (54 mmol) of triethylamine diluted by 15 mL of CH₂Cl₂ was added during 10 minutes and reaction mixture was stirred for 10 minutes at 0°C. The mixture was then heated slowly to 20-25°C and stirred for 1 h at this temperature. Solvents were distilled under reduced pressure and to the residue was added 170 mL of ethyl acetate. The obtained suspension was stirred for 10 minutes and the triethylamine hydrochloride solid was filtered. The filtrate was concentrated under reduced pressure. To the obtained residue 135 mL of ethyl acetate was added and suspension was heated to 40°C to dissolution. To obtained solution 135 mL of hexane was added during 20 minutes at 40°C and next cooled to 0°C during 30 minutes. Suspension was stirred for 30 minutes at 0°C. Solid was filtered and washed twice with 30 mL of hexane. Product was dried under reduced pressure to give 12 g of desired product. Yield: 49%.

### Example 6

### Sofosbuvir

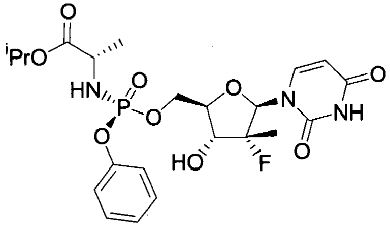

2'-Deoxy-2'-fluoro-2'-C-methyluridine 0.23 g (0.88 mmol) was dissolved in 10 ml of anhydrous THF and cooled to 0°C. 0.75 ml (0.97 mmol) of isopropylmagnesium chloride/lithium chloride complex (turbo Grignard) solution (1.3 M solution in THF) was added and the obtained suspension was stirred at 0°C for 10 minutes. Next, 0.8 g (1.76 mmol) of Oxyma-B intermediate (compound of formula la) was added and the reaction mixture was stirred for 2 h at 0°C, heated to 20-25°C over 2 h and stirred for 15 h at 20-25°C. After which 30 ml of ethyl acetate and 15 ml of saturated ammonium chloride solution were added. After stirring for 15 minutes the phases were separated. The organic phase was washed twice with 15 ml of saturated NaHCO₃ solution and with 20 ml of brine solution. The organic phase was then dried over anhydrous MgSO₄ and concentrated under reduced pressure. The product was obtained as a mixture of isomers (Sp:Rp isomers - 15:1). The isomeric ratio was determined by HPLC (150 x 4.6 mm Waters XBridge Shield RP-18 column, detection at 200-400nm, H₂O/MeCN gradient 90/10 to 10/90 at 20 minutes, Sp R_{T} = 10.3 min and Rp R_{T} = 10.1 min).

## Claims

1. A process for preparing a compound of formula I 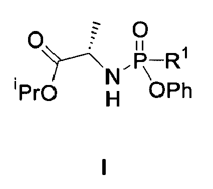 comprising reacting a compound of formula II 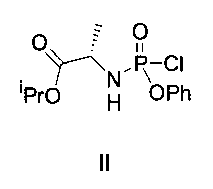 with a compound of formula IIIa, IIIb or IIIc 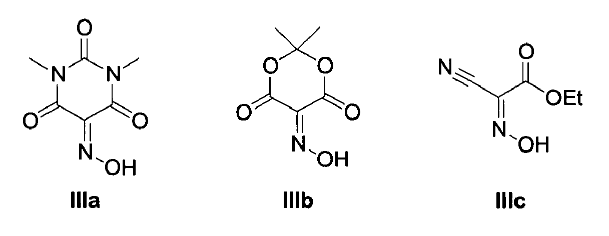 wherein R¹ represents:

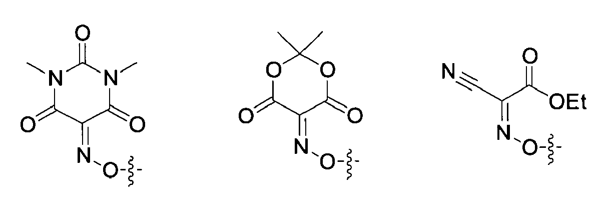

2. A process as claimed in claim 1, wherein a compound of formula II is reacted with a compound of formula IIIa.

3. A process as claimed in claim 2, wherein the reaction is conducted in the presence of a base and wherein the reaction is conducted at a temperature of from -10°C to 15°C.

4. A compound of formula la, or Ib, or Ic,

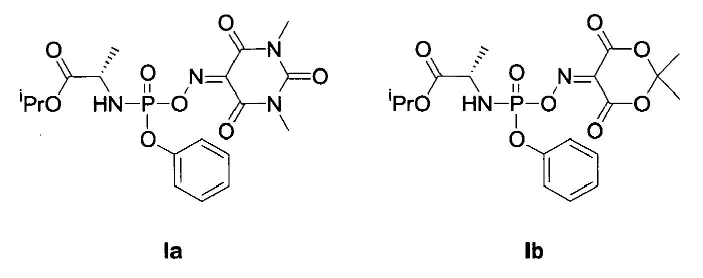

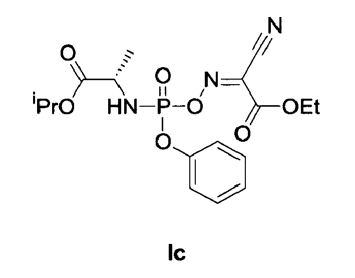

5. A process for preparing sofosbuvir, comprising reacting a compound of formula I with a compound of formula IV 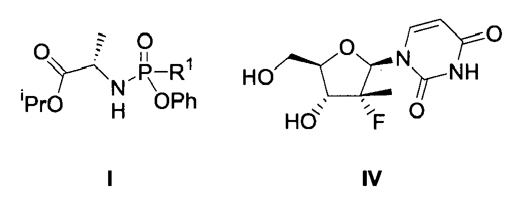 wherein R¹ is as defined hereinabove.

6. A process as claimed in claim 5, wherein the diastereomeric ratio is 10:1 or greater, in favour of sofosbuvir.

7. A process as claimed in claims 5 or 6, wherein the reaction is conducted at a temperature of from -40°C to 50°C.

8. A process as claimed in claim 7, wherein the reaction is conducted at a temperature of from -10°C to 0°C.

9. A process as claimed in any of claims 5 to 8, wherein the process further comprises preparing a compound of formula I by the process as claimed in claims 1, 2 or 3.

10. A process as claimed in any of claims 5 to 8, wherein the reaction of the compound of formula I with the compound of formula IV is performed in the presence of a base.

11. A process as claimed in claim 10, wherein the base is an alkyl magnesium-containing base.

12. A process as claimed in claim 11, wherein the base is selected from *tert*-butylmagnesium chloride, isopropylmagnesium chloride, methylmagnesium chloride, butylmagnesium chloride, isobutylmagnesium chloride or turbo Grignard.

13. A process as claimed in claim 12, wherein the base is turbo Grignard.

14. A process as claimed in claim 13, wherein the base is turbo Grignard and the diastereomeric ratio is 15:1 or greater, in favour of sofosbuvir.
